# EUROPEAN PATENT APPLICATION

(11) **EP 4 792 741 A1**
(43) Date of publication of application: **19.08.2026**
(21) Application number: 24877173.5
(22) Date of filing: 08.10.2024
(51) Int. Cl.: A61B 5/20

(54) **URINE FLOW RATE ESTIMATION DEVICE AND URINE FLOW RATE ESTIMATION PROGRAM**

(30) Priority: 10.10.2023 JP 2023175133
(71) Applicant: National University Corporation Saitama University, Saitama City, Saitama 338-8570 (JP); Saitama Medical University, Saitama 350-0495 (JP)
(72) Inventor: Kang, Donghyuk, Saitama-shi, Saitama 338-8570 (JP); Honma, Shunji, Saitama-shi, Saitama 338-8570 (JP); Kiyama, Akihito, Saitama-shi, Saitama 338-8570 (JP); Takeshita, Hideki, Iruma-gun, Saitama 350-0495 (JP)
(74) Representative: Ziebig Hengelhaupt Intellectual Property Attorneys Patentanwaltskanzlei PartGmbB
(86) International application number: PCT/JP2024/035947
(87) International publication number: WO 2025/079578

(57) **Abstract**

An object of the present invention is to improve the estimation accuracy of a urine flow rate. A urine flow rate estimation device 70 includes a camera 71 configured to acquire an image of discharged urine, an image analysis unit 72 configured to estimate a first urine flow rate based on the image acquired by the camera 71, a microphone 73 configured to acquire a sound associated with discharge of urine, a sound analysis unit 74 configured to estimate a second urine flow rate based on the sound acquired by the microphone 73, and a urine flow rate estimation unit 76 configured to estimate a third urine flow rate based on the first urine flow rate and the second urine flow rate.

## Description

### Technical Field

The present invention relates to a urine flow rate estimation device and a urine flow rate estimation program.

### Background Art

Uroflowmetry is essential in diagnosis, treatment, and follow-up observation of a urination disorder caused by prostatic hyperplasia, overactive bladder, neurogenic bladder, and the like. In medical institutions, it is common to use a method of recording urine volume and urine flow rate using a machine in which a cup is installed under a simple toilet bowl. However, since an enviro nment is different from a toilet used in daily life, a patient may become nervous or a timing of uri nation may not match, which often results in unsuccessful measurement. Moreover, although it is ideal to perform measurements a plurality of times in a daily state, it is not realistic to implement these methods at home from viewpoints of cost and hygiene.

In order to overcome such problems, a method of estimating the urine flow rate by analyzing a image captured during urination is disclosed (Patent Literature 1).

### Citation List

### Patent Literature

[Patent Literature 1] Japanese Unexamined Patent Application Publication No. 2018-109285

### Summary of Invention

### Technical Problem

However, since the technique disclosed in Patent Literature 1 estimates the urine flow rate only by image analysis during urination, there is room for improvement to improve estimation acc uracy of the urine flow rate.

Therefore, an object of one embodiment of the present invention is to improve the estimation accuracy of the urine flow rate.

### Solution to Problem

As one embodiment, the present application discloses a urine flow rate estimation device comprising: a camera configured to acquire an image of discharged urine; an image analysis unit configured to estimate a first urine flow rate based on the image acquired by the camera; a micro phone configured to acquire a sound associated with discharge of urine; a sound analysis unit con figured to estimate a second urine flow rate based on the sound acquired by the microphone; and a urine flow rate estimation unit configured to estimate a third urine flow rate based on the first urine flow rate and the second urine flow rate.

Also, as one embodiment, the present application discloses a urine flow rate estimation program for causing a processor to execute: a step of estimating a first urine flow rate based on an image of discharged urine acquired by a camera; a step of estimating a second urine flow rate based on a sound associated with discharge of urine acquired by a microphone; and a step of estimating a third urine flow rate based on the first urine flow rate and the second urine flow rate.

Also, as one embodiment, the present application discloses the urine flow rate estimation device, wherein the image analysis unit (the step of estimating the first urine flow rate) is configured to estimate the first urine flow rate based on a physical quantity correlated with an axis switching position of the discharged urine in the image of the discharged urine acquired by the camera.

Also, as one embodiment, the present application discloses the urine flow rate estimation device, wherein the physical quantity is a wavelength that is a distance between a maximum amplitude position forward of the axis switching position of the discharged urine and a urine discharge port in the image of the discharged urine acquired by the camera.

Also, as one embodiment, the present application discloses the urine flow rate estimation device, wherein the sound analysis unit (the step of estimating the second urine flow rate) is configured to estimate the second urine flow rate based on an energy of the sound acquired by the microphone.

Also, as one embodiment, the present application discloses the urine flow rate estimation device, wherein the urine flow rate estimation unit (the step of estimating the third urine flow rate) is configured to estimate the third urine flow rate using a missing data estimation method based on an eigenvalue decomposition of a matrix having the first urine flow rate, the second urine flow rate, and the third urine flow rate as components.

Also, as one embodiment, the present application discloses the urine flow rate estimation device, wherein the urine flow rate estimation unit (the step of estimating the third urine flow rate) is configured to, with respect to time-series data of the first urine flow rate and the second urine flow rate, estimate the second urine flow rate as the third urine flow rate for a region where the first urine flow rate is equal to or less than a first threshold, estimate the first urine flow rate as the third urine flow rate for a region where the first urine flow rate is greater than the first threshold and a frequency of the first urine flow rate is greater than a second threshold, and estimate, as the third urine flow rate, a urine flow rate based on the first urine flow rate and the second urine flow rate for a region where the first urine flow rate is greater than the first threshold and the frequency of the first urine flow rate is equal to or less than the second threshold.

Also, as one embodiment, the present application discloses a non-transitory computer-readable storage medium storing a urine flow rate estimation program for causing a processor to execute: a step of estimating a first urine flow rate based on an image of discharged urine acquired by a camera; a step of estimating a second urine flow rate based on a sound associated with discharge of urine, acquired by a microphone; and a step of estimating a third urine flow rate based on the first urine flow rate and the second urine flow rate.

According to one embodiment of the present invention, the estimation accuracy of the urine flow rate can be improved.

### Brief Description of Drawings

FIG. 1 is a diagram schematically illustrating an experimental apparatus including the urine flow rate estimation device of the present embodiment.
FIG. 2 is a diagram schematically illustrating functional blocks of the urine flow rate estimation device.
FIG. 3 is a diagram schematically illustrating a shape of a nozzle that discharges urine.
FIG. 4A is a diagram schematically illustrating a background image when an image of the urine discharged from the nozzle is captured from a side.
FIG. 4B is a diagram schematically illustrating an image captured from the side of the urine discharged from the nozzle.
FIG. 5 is a diagram schematically illustrating a correlation between a wavelength change and the urine flow rate.
FIG. 6A is a diagram illustrating an example of image processing for suppressing erroneous estimation caused by splashing.
FIG. 6B is a diagram illustrating an example of the image processing for suppressing the erroneous estimation caused by the splashing.
FIG. 7A is a diagram schematically illustrating an energy density of a sound for each frequency over time.
FIG. 7B is a diagram schematically illustrating an energy of the sound over time.
FIG. 7C is a diagram schematically illustrating a smoothed energy of the sound over time.
FIG. 8 is a diagram illustrating an estimation result (No. 1) of the urine flow rate.
FIG. 9 is a diagram illustrating an estimation result (No. 2) of the urine flow rate.
FIG. 10 is a diagram illustrating an estimation result (No. 3) of the urine flow rate.
FIG. 11 is a diagram illustrating an estimation result (No. 4) of the urine flow rate.
FIG. 12 is a diagram illustrating an estimation result (No. 5) of the urine flow rate.
FIG. 13 is a diagram illustrating errors of the estimation results (Nos. 1 to 5) obtained by various estimation methods.
FIG. 14 is a diagram illustrating an example of a urine flow rate estimation performed by the urine flow rate estimation unit.
FIG. 15 is a flowchart for explaining the urine flow rate estimation program of the present embodiment.

### Description of Embodiments

Hereinafter, the urine flow rate estimation device and the urine flow rate estimation program of the present embodiment will be described with reference to the drawings.

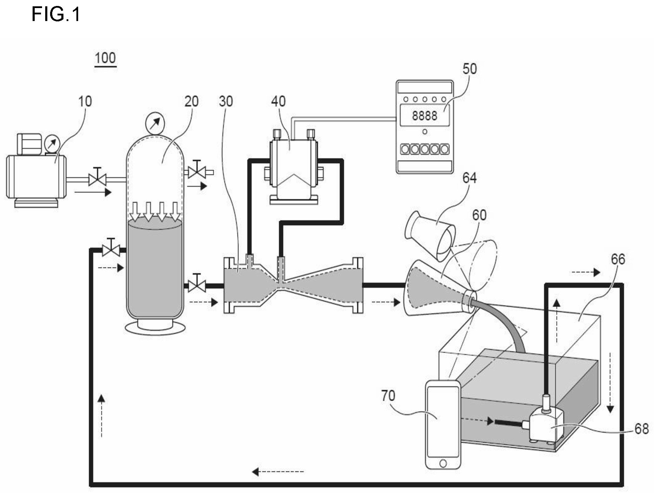

FIG. 1 is a diagram schematically illustrating an experimental apparatus including the urine flow rate estimation device of the present embodiment. The experimental apparatus 100 is configured to force a liquid simulating urine (hereinafter, simply referred to as urine) stored in a tank 20 using a compressor 10, and to discharge the urine from a nozzle 60 via a Venturi tube 30. A differential pressure transducer 40 is connected to the Venturi tube 30. The differential pressure transducer 40 is configured to calculate the urine flow rate based on a difference in pressure between two different locations of the Venturi tube 30, using Bernoulli's theorem. The calculated urine flow rate is displayed on a monitor 50. In the present specification, the urine flow rate refers to a urine flow rate (ml/s) per unit time.

The urine discharged from the nozzle 60 is stored in a container 66, and is then returned to the tank 20 by a pump 68. A light source 64 is disposed in the vicinity of the nozzle 60 so that an image of the urine discharged from the nozzle 60 can be clearly acquired.

In the present embodiment, an example in which a smartphone that can be held and used by a patient is used as the urine flow rate estimation device 70 is shown, but the present invention is not limited thereto. The urine flow rate estimation device 70 may be any device having functions described below.

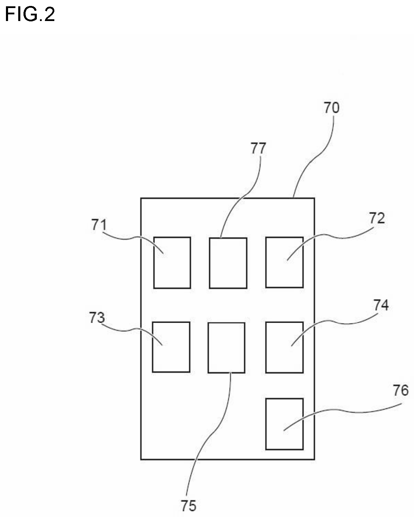

FIG. 2 is a diagram schematically illustrating functional blocks of the urine flow rate estimation device. As illustrated in FIG. 2, the urine flow rate estimation device 70 includes a camera 71 configured to acquire the image of the discharged urine, a microphone 73 configured to acquire the sound associated with the discharge of the urine, a memory 75 storing the urine flow rate estimation program, and a processor 77 configured to read and execute the urine flow rate estimation program stored in the memory 75.

The urine flow rate estimation device 70 includes, as functional blocks realized by the processor 77 executing the urine flow rate estimation program, an image analysis unit 72 configured to estimate the first urine flow rate based on the image acquired by the camera 71, a sound analysis unit 74 configured to estimate the second urine flow rate based on the sound acquired by the microphone 73, and the urine flow rate estimation unit 76 configured to estimate the third urine flow rate based on the first urine flow rate and the second urine flow rate.

The urine flow rate estimation program may be downloaded to a memory of a device such as a smartphone via a communication network such as the Internet and executed, or may be stored in a non-transitory storage medium readable by a processor and sold.

The camera 71 only needs to be capable of acquiring the image with such accuracy that the axis switching position of the urine can be recognized when the image of the discharged urine is acquired from a side. In the present embodiment, an example in which the image of the discharged urine is acquired from the side is shown, but the present invention is not limited thereto, and the image of the discharged urine can be acquired from any direction. The camera 71 only needs to be capable of acquiring the image with such accuracy that the axis switching position of the urine can be recognized when the image of the discharged urine is acquired from any direction.

That is, the present embodiment applies an axis switching phenomenon, which is one of interfacial instability phenomena of a fluid. Axis switching is a phenomenon in which, when a liquid flows out from a nozzle having an elliptical cross-sectional shape rather than a circular cross-sectional shape, a major axis and a minor axis of an elliptical liquid column cross section shown immediately after an outflow are interchanged, and a jet having a shape like a twisted bar is observed (see Rayleigh, L., "On the Capillary Phenomena of Jets," Proc. R. Soc. London, 21, 71-97, (1879)). Since a shape of a distal end of a urethra is generally an ellipse, the axis switching also occurs in the discharged urine.

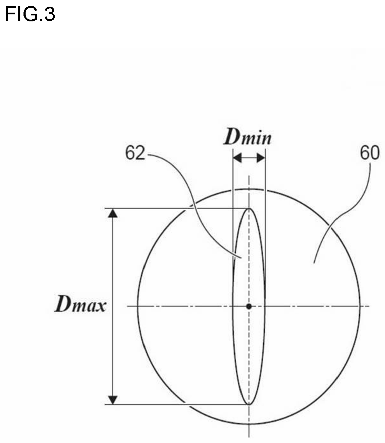

FIG. 3 is a diagram schematically illustrating the shape of the nozzle that discharges the urine. As illustrated in FIG. 3, a discharge port 62 of the nozzle 60 has a vertically elongated elliptical shape simulating the shape of the distal end of the urethra. A ratio between a minor axis (Dmin) and a major axis (Dmax) of the elliptical shape of the discharge port 62 is 1:6.

The image analysis unit 72 is configured to estimate the first urine flow rate based on a physical quantity correlated with the axis switching position of the discharged urine in the image acquired by the camera 71.

In the present embodiment, specifically, the image analysis unit 72 adopts a wavelength that is a distance between a maximum amplitude position forward of the axis switching position of the discharged urine and the urine discharge port in the image acquired by the camera 71, as the physical quantity correlated with the axis switching position of the discharged urine. Hereinafter, this point will be described.

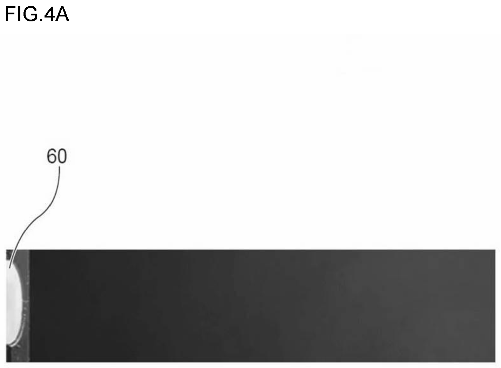

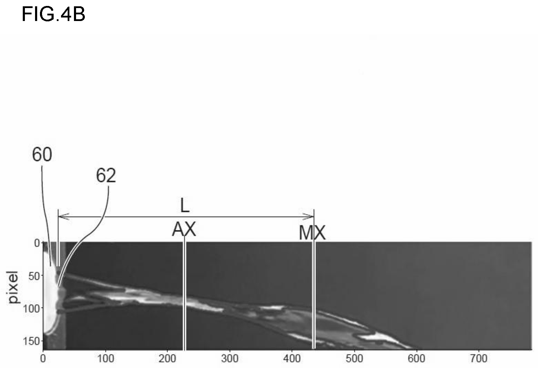

FIG. 4A is a diagram schematically illustrating a background image when the urine discharged from the nozzle is acquired from the side. FIG. 4B is a diagram schematically illustrating an image acquired from the side of the urine discharged from the nozzle.

As illustrated in FIG. 4B, the image analysis unit 72 extracts an outline of the urine by binarizing the image acquired by the camera 71 using an existing image analysis method, and obtains an axis switching position (a minimum amplitude position of the urine) AX. Further, the image analysis unit 72 obtains a maximum amplitude position MX of the urine forward of the axis switching position AX. The image analysis unit 72 obtains a wavelength L that is a distance between the urine discharge port (the discharge port 62 of the nozzle) and the maximum amplitude position MX.

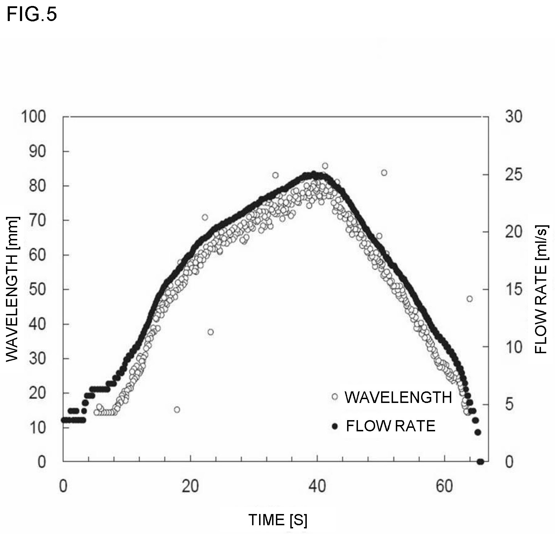

The image analysis unit 72 estimates the first urine flow rate based on the wavelength L in the image acquired by the camera 71. FIG. 5 is a diagram schematically illustrating a correlation between a wavelength change and the urine flow rate. In FIG. 5, a horizontal axis represents time (s), a vertical axis (left) represents a wavelength (mm), and a vertical axis (right) represents a urine flow rate (ml/s). In FIG. 5, the wavelength is plotted with white circles, and the urine flow rate is plotted with black circles. As illustrated in FIG. 5, there is a correlation between the wavelength and the urine flow rate.

The image analysis unit 72 can estimate the first urine flow rate based on the wavelength L in the image acquired by the camera 71 by obtaining a conversion formula from the wavelength L (mm) to the urine flow rate (ml/s) in advance through an experiment, a simulation, or the like. In the present embodiment, an example in which the image analysis unit 72 estimates the first urine flow rate based on the wavelength L has been shown, but the present invention is not limited thereto, and the first urine flow rate can also be estimated based on a distance between the urine discharge port (the discharge port 62 of the nozzle) and the axis switching position AX.

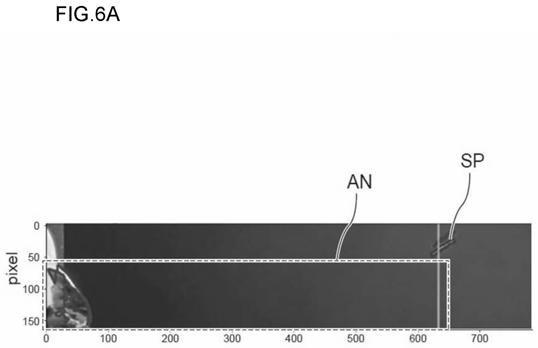

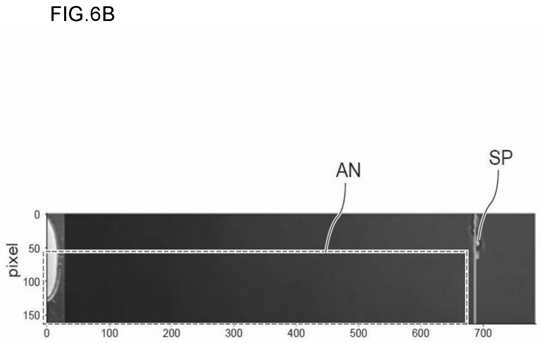

Note that the image analysis unit 72 can set an image analysis region in order to suppress occurrence of erroneous estimation caused by splashing of liquid resulting from the discharged urine. FIG. 6A and FIG. 6B are diagrams illustrating an example of the image processing for suppressing the erroneous estimation caused by the splashing.

When the urine discharged from the nozzle 60 lands in the container 66 (a toilet bowl in actual use), the discharged urine itself may hit the container 66 and splash back, or a liquid such as water or urine accumulated in the container 66 may splash back, which may be captured as a splash SP in the image acquired by the camera 71 as illustrated in FIG. 6A and FIG. 6B. In this case, the image analysis unit 72 may erroneously detect the splashing SP as the maximum amplitude position MX, and as a result, may erroneously estimate the first urine flow rate.

Therefore, the image analysis unit 72 can set a region through which the discharged urine passes among the entire region of the image acquired by the camera 71 as an image analysis region AN. In this case, the image analysis unit 72 obtains the maximum amplitude position MX and the wavelength L based on an image analysis within the image analysis region AN, thereby making it possible to suppress the occurrence of the erroneous estimation caused by the splashing.

The microphone 73 only needs to be configured to acquire the sound associated with the discharge of the urine (for example, a sound when the urine lands in the container 66). The sound analysis unit 74 is configured to estimate the second urine flow rate based on an energy of the sound acquired by the microphone 73. That is, there is a correlation between the energy (loudness) of the sound associated with the discharged urine and the urine flow rate. Therefore, the sound analysis unit 74 can estimate the second urine flow rate based on the energy of the sound acquired by the microphone 73 using an existing sound analysis method.

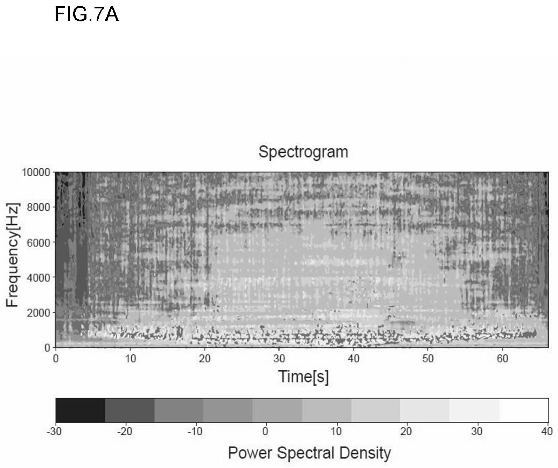

FIG. 7A is a diagram schematically illustrating an energy density of a sound for each frequency over time. In FIG. 7A, a horizontal axis represents time (s), and a vertical axis represents frequency (Hz). FIG. 7A illustrates a power spectral density for each frequency of the sound caused by the urine in grayscale. The sound analysis unit 74 can obtain the power spectral density for each frequency over time as illustrated in FIG. 7A using the existing sound analysis method. In addition, the sound analysis unit 74 can also remove noise of an environmental sound such as a sound of a toilet fan using the existing sound analysis method.

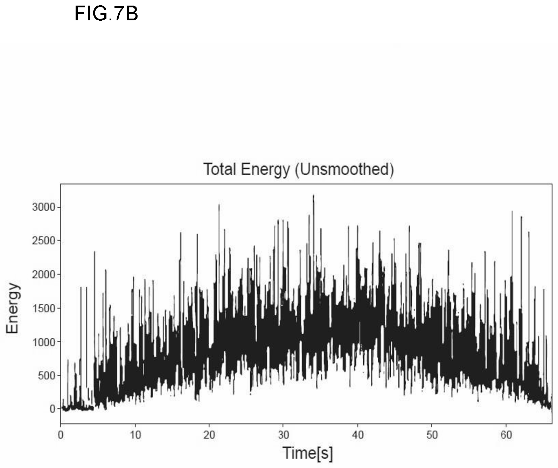

FIG. 7B is a diagram schematically illustrating an energy of the sound over time. In FIG. 7B, a horizontal axis represents time (s), and a vertical axis represents an energy of a sound. The sound analysis unit 74 can obtain a total energy of the sound over time (a total energy of all frequencies) as illustrated in FIG. 7B using the existing sound analysis method.

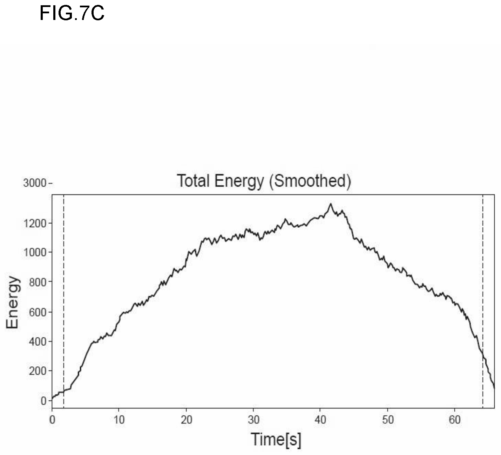

FIG. 7C is a diagram schematically illustrating a smoothed energy of the sound over time. In FIG. 7C, a horizontal axis represents time (s), and a vertical axis represents an energy of a sound. The sound analysis unit 74 can obtain the smoothed energy of the sound over time as illustrated in FIG. 7C by, for example, averaging data of a plurality of points (for example, 150 points) before and after a measurement point to smooth a graph of FIG. 7B. The sound analysis unit 74 can estimate the second urine flow rate based on the energy of the sound acquired by the microphone 73 by obtaining a conversion formula from the energy of the sound to the urine flow rate (ml/s) in advance through an experiment, a simulation, or the like.

The urine flow rate estimation unit 76 is configured to estimate the third urine flow rate based on the first urine flow rate and the second urine flow rate. Specifically, the urine flow rate estimation unit 76 is configured to estimate the third urine flow rate using a missing data estimation method based on an eigenvalue decomposition of a matrix having the first urine flow rate, the second urine flow rate, and the third urine flow rate as components. In the present embodiment, a Gappy POD is used as an example of the missing data estimation method based on the eigenvalue decomposition. Hereinafter, an estimation of the third urine flow rate using the Gappy POD will be described.

A sparse singular value decomposition (Gappy POD) is a type of singular value decomposition of a matrix. The singular value decomposition decomposes a given matrix into three matrices, and the decomposed three matrices can represent a correlation between row and column elements and a strength thereof, respectively. First, after constructing complete training data, the singular value decomposition is performed. An eigenvector obtained by the singular value decomposition represents a correlation between measured parameters (sound and wavelength) and the urine flow rate, and the urine flow rate (the third urine flow rate) can be estimated using the correlation vector. In particular, the sparse singular value decomposition (Gappy POD) can easily obtain a correlation among a large number of parameters, and can estimate the urine flow rate even if not all data are measured. Specifically, in a place where it is not easy to capture an image of the discharged urine or when a sound associated with the discharge of the urine cannot be acquired due to a large environmental noise, even if complete data (sound and image) cannot be acquired, a urine flow rate can be estimated using only either one of the data.

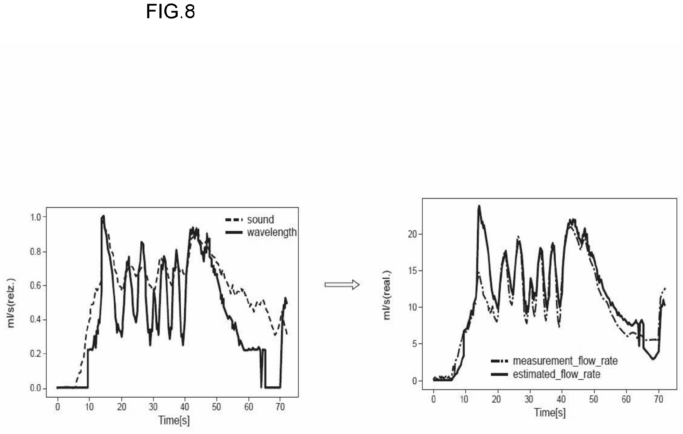

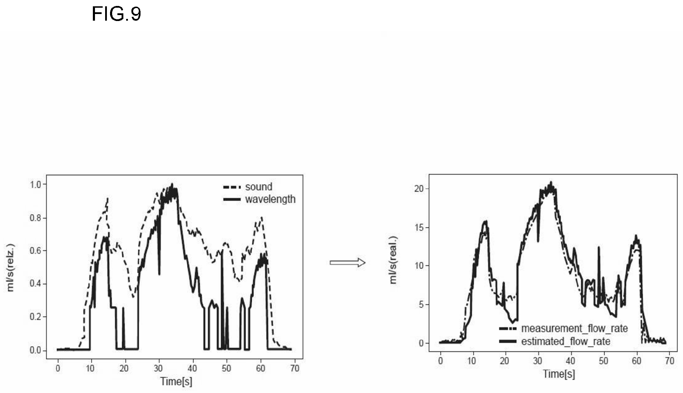

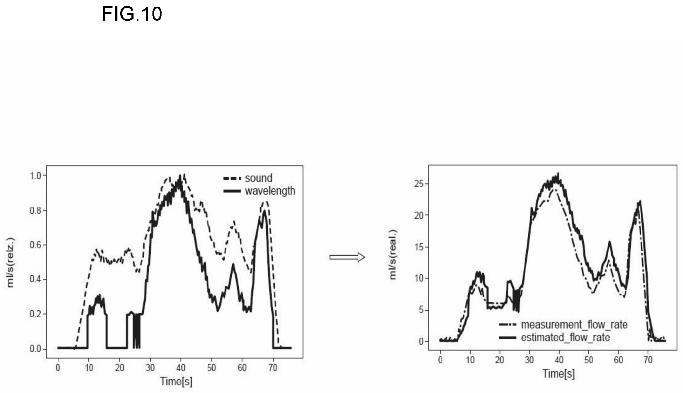

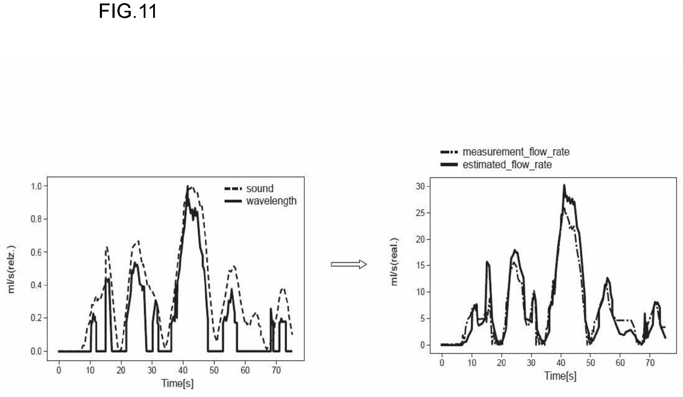

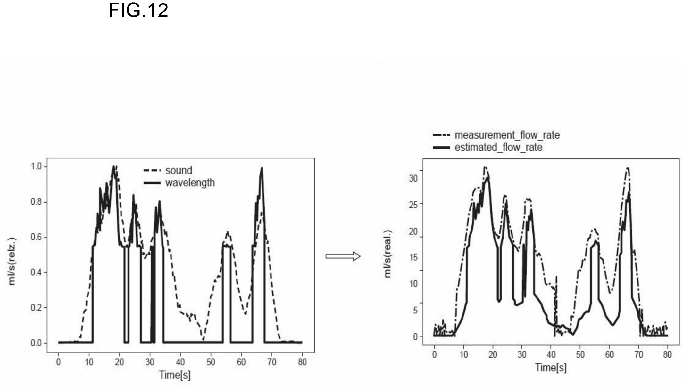

FIG. 8 to FIG. 12 are diagrams illustrating estimation results (Nos. 1 to 5) of the urine flow rate. FIG. 8 to FIG. 12 illustrate the urine flow rate estimation results for five different types of urine discharge patterns. In graphs on the left side of FIG. 8 to FIG. 12, a horizontal axis represents time (s), and a vertical axis represents the normalized first urine flow rate (solid line) and the second urine flow rate (broken line). In graphs on the right side of FIG. 8 to FIG. 12, a horizontal axis represents time (s), and a vertical axis represents the third urine flow rate (ml/s, solid line) and the urine flow rate (ml/s, broken line) measured using the differential pressure transducer 40.

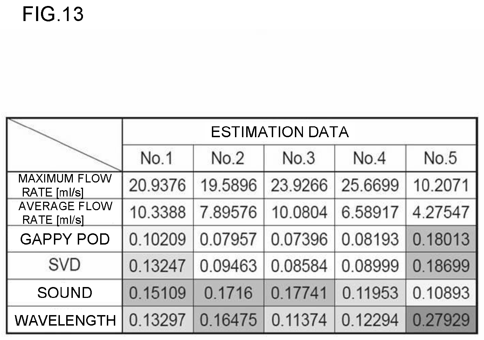

FIG. 13 is a diagram illustrating errors of the estimation results (Nos. 1 to 5) obtained by various estimation methods. FIG. 13 illustrates RMSEs (root mean square errors) of the estimation results of the urine flow rate estimation based on the Gappy POD, a urine flow rate estimation based on SVD (singular value decomposition), a urine flow rate estimation based only on the sound analysis, and a urine flow rate estimation based only on the image analysis (wavelength L). A smaller RMSE means a smaller error. As illustrated in FIG. 13, it can be seen that by performing the urine flow rate estimation based on the Gappy POD, the accuracy of the urine flow rate estimation is improved compared to other estimation methods.

According to the present embodiment, the estimation accuracy of the urine flow rate can be improved by estimating the urine flow rate based on estimation results of both an image analysis and a sound analysis during urination, instead of estimating the urine flow rate only by the image analysis during urination. Moreover, according to the present embodiment, since the urine flow rate is estimated using the missing data estimation method based on the eigenvalue decomposition, the urine flow rate can be accurately estimated even when missing data occurs in image data or sound data.

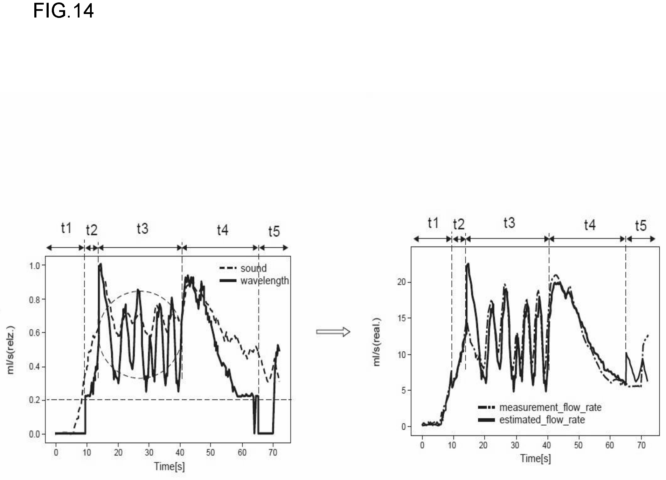

In the above-described embodiment, the example in which the urine flow rate estimation unit 76 estimates the third urine flow rate using the missing data estimation method based on the eigenvalue decomposition has been shown, but the present invention is not limited thereto. FIG. 14 is a diagram illustrating an example of a urine flow rate estimation performed by the urine flow rate estimation unit.

The urine flow rate estimation unit 76 can, with respect to time-series data of the first urine flow rate and the second urine flow rate, estimate the second urine flow rate as the third urine flow rate for regions (regions of t1 and t5) where the first urine flow rate is equal to or less than a first threshold (for example, 0.2 ml/s). Further, the urine flow rate estimation unit 76 can, with respect to the time-series data of the first urine flow rate and the second urine flow rate, estimate the first urine flow rate as the third urine flow rate for a region (a region of t3) where the first urine flow rate is greater than the first threshold and a frequency of the first urine flow rate is greater than a second threshold.

Furthermore, the urine flow rate estimation unit 76 can, with respect to the time-series data of the first urine flow rate and the second urine flow rate, estimate, as the third urine flow rate, a urine flow rate based on the first urine flow rate and the second urine flow rate for a region (regions of t2 and t4) where the first urine flow rate is greater than the first threshold and the frequency of the first urine flow rate is equal to or less than the second threshold.

For example, for the regions of t2 and t4, the urine flow rate estimation unit 76 may estimate an average value of the first urine flow rate and the second urine flow rate as the third urine flow rate. Further, for example, for the regions of t2 and t4, the urine flow rate estimation unit 76 may estimate the third urine flow rate using a missing data estimation method based on an eigenvalue decomposition of a matrix having the first urine flow rate, the second urine flow rate, and the third urine flow rate as components.

According to the present embodiment, it is possible to perform a highly accurate estimation of the urine flow rate in consideration of characteristics of both the estimation of the urine flow rate based on the image and the estimation of the urine flow rate based on the sound. That is, the inventors of the present application have found that the estimation of the urine flow rate based on the image analysis cannot be performed when a flow rate of the discharged urine is low (the estimation result becomes almost zero although there is an actual flow rate), as shown in the regions of t1 and t5 in FIG. 14. On the other hand, the inventors of the present application have found that the estimation of the urine flow rate based on the sound analysis can estimate an entire flow rate, but estimation accuracy is poor when there is small flow rate fluctuations (when a frequency of the flow rate is high) as shown in the region of t3 in FIG. 14.

Therefore, for the regions (the regions of t1 and t5) where the first urine flow rate is low, since reliability of the urine flow rate (the first urine flow rate) based on the image analysis is low, the urine flow rate estimation unit 76 adopts the urine flow rate (the second urine flow rate) based on the sound analysis as the third urine flow rate. Further, for the region (the region of t3) where there is a fine flow rate fluctuation in the first urine flow rate, since reliability of the urine flow rate (the second urine flow rate) based on the sound analysis is low, the urine flow rate estimation unit 76 adopts the urine flow rate (the first urine flow rate) based on the image analysis as the third urine flow rate.

Furthermore, for a region (the regions of t2 and t4) corresponding to neither of the above, since reliabilities of both the urine flow rate (the first urine flow rate) based on the image analysis and the urine flow rate (the second urine flow rate) based on the sound analysis are high, the urine flow rate estimation unit 76 estimates the third urine flow rate based on both of them. As a result, the urine flow rate estimation unit 76 can improve the estimation accuracy of the urine flow rate by performing the estimation of the urine flow rate in consideration of the characteristics of both the estimation of the urine flow rate based on the image analysis and the estimation of the urine flow rate based on the sound analysis.

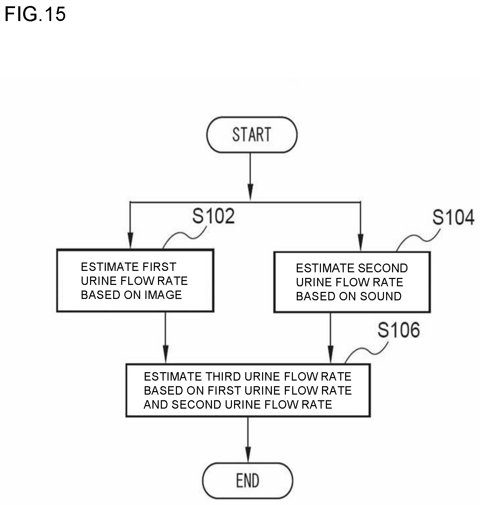

Next, the urine flow rate estimation program of the present embodiment will be described. FIG. 15 is a flowchart for explaining the urine flow rate estimation program of the present embodiment.

As illustrated in FIG. 15, the urine flow rate estimation program causes the processor (the processor 77) to execute step 102 of estimating the first urine flow rate based on the image of the discharged urine acquired by the camera (for example, the camera 71).

Step 102 of estimating the first urine flow rate is configured to estimate the first urine flow rate based on a physical quantity correlated with an axis switching position of the discharged urine in an image of the discharged urine acquired from the side by the camera, similarly to the processing performed by the above-described image analysis unit 72. The physical quantity may be a wavelength that is a distance between a maximum amplitude position forward of the axis switching position of the discharged urine and a urine discharge port in the image of the discharged urine acquired from the side by the camera.

Further, in parallel with step 102, the urine flow rate estimation program causes the processor (the processor 77) to execute step 104 of estimating the second urine flow rate based on the sound associated with the discharge of the urine, acquired by the microphone (for example, the microphone 73).

Step 104 of estimating the second urine flow rate is configured to estimate the second urine flow rate based on the energy of the sound acquired by the microphone, similarly to the processing performed by the above-described sound analysis unit 74.

Further, the urine flow rate estimation program causes the processor (the processor 77) to execute step 106 of estimating the third urine flow rate based on the first urine flow rate estimated in step 102 and the second urine flow rate estimated in step 104.

Step 106 of estimating the third urine flow rate is configured to estimate the third urine flow rate using a missing data estimation method based on an eigenvalue decomposition of a matrix having the first urine flow rate, the second urine flow rate, and the third urine flow rate as components, similarly to the processing performed by the above-described urine flow rate estimation unit 76.

Not limited to this, step 106 of estimating the third urine flow rate may, with respect to time-series data of the first urine flow rate and the second urine flow rate, estimate the second urine flow rate as the third urine flow rate for a region where the first urine flow rate is equal to or less than a first threshold, estimate the first urine flow rate as the third urine flow rate for a region where the first urine flow rate is greater than the first threshold and a frequency of the first urine flow rate is greater than a second threshold, and estimate, as the third urine flow rate, a urine flow rate based on the first urine flow rate and the second urine flow rate for a region where the first urine flow rate is greater than the first threshold and the frequency of the first urine flow rate is equal to or less than the second threshold.

According to the urine flow rate estimation program of the present embodiment, the estimation accuracy of the urine flow rate can be improved similarly to the above-described urine flow rate estimation device 70.

### Reference Signs List

60 nozzle
62 discharge port
70 urine flow rate estimation device
71 camera
72 image analysis unit
73 microphone
74 sound analysis unit
75 memory
76 urine flow rate estimation unit
77 processor
AX axis switching position
L wavelength
MX maximum amplitude position

## Claims

1. A urine flow rate estimation device comprising:
a camera configured to acquire an image of discharged urine;
an image analysis unit configured to estimate a first urine flow rate based on the image acquired by the camera;
a microphone configured to acquire a sound associated with discharge of urine;
a sound analysis unit configured to estimate a second urine flow rate based on the sound acquired by the microphone; and
a urine flow rate estimation unit configured to estimate a third urine flow rate based on the first urine flow rate and the second urine flow rate.

2. The urine flow rate estimation device according to claim 1, wherein
the image analysis unit is configured to estimate the first urine flow rate based on a physical quantity correlated with an axis switching position of the discharged urine in the image of the discharged urine acquired by the camera.

3. The urine flow rate estimation device according to claim 2, wherein
the physical quantity is a wavelength that is a distance between a maximum amplitude position forward of the axis switching position of the discharged urine and a urine discharge port in the image of the discharged urine acquired by the camera.

4. The urine flow rate estimation device according to claim 3, wherein
the sound analysis unit is configured to estimate the second urine flow rate based on an energy of the sound acquired by the microphone.

5. The urine flow rate estimation device according to any one of claims 1 to 4, wherein
the urine flow rate estimation unit is configured to estimate the third urine flow rate using a missing data estimation method based on an eigenvalue decomposition of a matrix having the first urine flow rate, the second urine flow rate, and the third urine flow rate as components.

6. The urine flow rate estimation device according to any one of claims 1 to 4, wherein
the urine flow rate estimation unit is configured to, with respect to time-series data of the first urine flow rate and the second urine flow rate, estimate the second urine flow rate as the third urine flow rate for a region where the first urine flow rate is equal to or less than a first threshold, estimate the first urine flow rate as the third urine flow rate for a region where the first urine flow rate is greater than the first threshold and a frequency of the first urine flow rate is greater than a second threshold, and estimate, as the third urine flow rate, a urine flow rate based on the first urine flow rate and the second urine flow rate for a region where the first urine flow rate is greater than the first threshold and the frequency of the first urine flow rate is equal to or less than the second threshold.

7. A urine flow rate estimation program for causing a processor to execute:
a step of estimating a first urine flow rate based on an image of discharged urine acquired by a camera;
a step of estimating a second urine flow rate based on a sound associated with discharge of urine, acquired by a microphone; and
a step of estimating a third urine flow rate based on the first urine flow rate and the second urine flow rate.
